# EUROPEAN PATENT APPLICATION

(11) **EP 2 078 713 A1**
(43) Date of publication of application: **15.07.2009**
(21) Application number: 07150479.9
(22) Date of filing: 28.12.2007
(51) Int. Cl.: C07D 211/60, C07D 231/40, A61K 31/445, A61P 31/04

(54) **Inhibitors of biofilm formation of gram-positive and gram-negative bacteria**

(71) Applicant: QuoNova GmbH, 82152 Martinsried (DE)
(72) Inventor: Ammendola, Aldo, 80638, München (DE); Wieber, Tanja, 82239, Alling (DE); Wuzik, Andreas, 86836, Untermeitingen (DE); Lang, Martin, 82166, Gräfelfing (DE)
(74) Representative: Vossius & Partner

(57) **Abstract**

The present invention relates to the use of compounds as broad spectrum inhibitors of bacterial biofilm formation. In particular the invention refers to a family of compounds that block the quorum sensing system of Gram-negative and Gram-positive bacteria, a process for their manufacture, pharmaceutical compositions containing them and to their use for the treatment and prevention of bacterial damages and diseases, in particular for diseases where there is an advantage in inhibiting quorum sensing regulated phenotypes of pathogens.

## Description

### Background of the invention

The present invention relates to the use of compounds as broad spectrum inhibitors of bacterial biofilm formation. In particular the invention refers to a family of compounds that block the quorum sensing system of Gram-negative and Gram-positive bacteria, a process for their manufacture, pharmaceutical compositions containing them and to their use for the treatment and prevention of bacterial damages and diseases, in particular for diseases where there is an advantage in inhibiting quorum sensing regulated phenotypes of pathogens.

Extracellular autoinducing compounds in the supernatants of microbial cultures were first recognized for their roles in the induction of genetic competence in gram-positive bacteria and in the regulation of light production in marine *Vibrio* species. "Quorum sensing" enables bacterial cells to chemically measure the density of the surrounding population. Subsequently, many examples of cell density-dependent gene regulation by extracellular signal molecules have been found in diverse microorganisms. The widespread incidence of diverse quorum sensing systems strongly suggests that regulation in accordance with cell density is important for the success of microbes in many environments. Cell density-dependent regulatory networks in microorganisms generally control processes that involve cell-cell interactions, such as group motility and the formation of multicellular structures. In a wide array of environmental and medically relevant bacteria, the development, maintenance, and dispersion of multicellular, surface-associated biofilms are in part controlled by quorum sensing regulatory pathways. The uptake of extracellular DNA is often regulated in accordance with cell density presumably to enhance the chances of taking up DNA from closely related strains. For some bacteria, a link between the competence and biofilm formation has been established. Because quorum sensing has been implicated as an important factor in the expression of virulence genes in human, animal and plant pathogens, including the formation of biofilms, quorum sensing blocking agents show promise for a novel antifouling and anti-microbial approach. Many microorganisms, including bacteria, fungi, protozoa and algae cause severe damages or diseases in different areas such as industry, agriculture, environment and medicine. Bacteria as human pathogens in particular cause tremendous costs in public health systems worldwide. The continuing emergence of multiple-drug-resistant bacterial strains has necessitated finding new compounds that can be used in antibacterial treatment. There are two broad strategies for the control of bacterial infection: Either to kill the organism or to attenuate its virulence such that it fails to adapt to the host environment. The latter approach has, however, lacked specific targets for rational drug design. The discovery that bacteria employ signal transduction pathways comprising small molecules to globally regulate the production of virulence determinants offers such a novel target.

A wide variety of Gram-negative bacteria produce N-acyl-L-homoserine lactone (HSL) derivatives as signal molecules in intercellular communication. These molecules, also referred to as "pheromones" or "quoromones", comprise a homo-serine lactone moiety linked to an acyl side chain. Bacteria use this signaling system to monitor their population cell density by quorum sensing. In each cell of a population an HSL synthase from usually the LuxI family of proteins produces a low basal level of diffusible HSLs. The HSL concentration increases with bacterial population density until a threshold concentration is reached which results in expression of various HSL-dependent genes through an HSL-receptor protein belonging generally to the LuxR family of transcriptional regulators. This HSL-receptor protein complex serves not only as positive transcription regulator of quorum sensing regulated genes but also as positive regulator for the HSL synthesis itself. Therefore, the entire system is amplified via a process of autoinduction.

This system was first discovered in the bioluminescent marine bacteria *Vibrio harveyi* and *V*. *fischer* where it is used to control bioluminescence expression. In recent years it has become apparent that many other Gram-negative bacteria employ one or more quorum sensing systems comprising HSL derivatives with different acyl side chains to regulate, in a cell-density dependent manner, a wide variety of physiological processes such as swarming motility, biofilm formation, pathogenicity, conjugation, bioluminescence or production of pigments and antibiotics (for reviews and further references see, e. g.: Fuqua et al., Ann. Rev. Microbiol. 50: 727-51, 1996; Fuqua & Greenberg, Curr. Opinion Microbiol. 1:183-89, 1998; Eberl, Syst. Appl. Microbiol. 22: 493-506, 1999; De Kievit & Iglewski, Infect. Immun. 68: 4839-49, 2000).

With regard to bacteria that utilize HSL-based quorum sensing as part of their lifestyle, *Pseudomonas aeruginosa* is perhaps the best understood in terms of the role quorum sensing plays in pathogenicity. In this human opportunistic pathogen, which causes nosocomial infections in immunocompromized patients and has an extremely high potential to develop resistance mechanisms against traditional antibiotic treatment, production of many virulence factors including expression of alkaline protease, endoproteinase, LasA protease, LasB elastase, anthranilate synthase, hemolysins, lectin, cytochrome c oxidase, catalase, Mn-and Fe-dependent superoxide dismutases, exotoxin A, exoenzyme S, chitinase, chitin binding protein, phenazine, hydrogen cyanide, pyocyanin, pyoverdine, phospholipase C, rhamnolipids, sigma factor S, components of the protein secretion apparatus, efflux transporters, production of alginate and adhesins, twitching motility and pilin export is regulated by two interlinked quorum sensing circuits. Moreover, it has been demonstrated that this signaling system is involved in the ability of *P. aeruginosa* to form biofilms (Davies et al., Science 280: 295-8, 1998). Huber et al. (Microbiology 147: 2517-28, 2001) demonstrated that biofilm formation and swarming motility of *Burkhoderia cepacia*, like *P. aeruginosa* a human opportunistic pathogen, is also dependent on a HSL-based quorum sensing system.

Gram-positive bacteria like staphylococci have developed different quorum-sensing systems that enable cell-to-cell communication and regulation of numerous colonization and virulence factors. The staphylococcal accessory gene regulator (*agr*) quorum sensing system decreases the expression of several cell surface proteins and increases the expression of many secreted virulence factors in the transition from late-exponential growth to stationary phase *in vitro.* Expression of *agr* was found to contribute to staphylococcal pathogenesis in several infection models, including murine subcutaneous abscesses and arthritis, as well as rabbit endocarditis. Expression of *agr* also appears to be involved in the invasion and apoptosis of epithelial cells. Two primary transcripts, RNAII and RNAIII, are generated by the *agr* locus and originate from the P2 and P3 promoters, respectively. The P2 operon encodes four proteins that generate the *agr*-sensing mechanism. AgrB is a transmembrane protein that appears to be involved in (a) processing of the *agrD* product into an octapeptide; (b) secretion of the autoinducing peptide (AIP) signal; and (c) modification of the AIP by the formation of a cyclic thiolactone bond between an internal cysteine and the carboxyl terminus. AgrA and AgrC form a two-component regulatory system in which the transmembrane component, AgrC (histidine kinase), binds the extracellular AIP and in turn modulates the activity of AgrA, the response regulator. Through an as-yet-undefined mechanism, AgrA activity then leads to greatly increased P2 and P3 transcription in the late-log phase of growth, when the concentration of the signal in the medium is high. Sequence variation in *agrB, agrD*, and *agrC* has led to the identification of at least four *S. aureus agr* specificity groups in which AIP produced by one group inhibits *agr* expression in other groups (for a review see: Yarwood and Schlievert, J. Clin. Invest. 112: 1520-1625, 2003).

*Staphylococcus aureus* and *Staphylococcus epidermidis* normally colonize the epithelial surfaces of large numbers of humans. *S. epidermidis* is considered part of the normal human microbial flora, while *S*. *aureus* is usually regarded as a transient member. Colonization by either species usually does not lead to adverse events. However, when these organisms or their extracellular products are allowed to breach the epithelial layer, serious disease can result. *S*. *aureus* has many cell surface virulence factors (such as protein A and clumping factor) and secreted exotoxins and enzymes that allow strains to cause a myriad of infections. These diseases range from relatively benign furuncles and subcutaneous abscesses to scalded skin syndrome, sepsis, necrotizing pneumonia, and toxic shock syndrome (TSS). While no single cell surface virulence factor has been shown to be uniquely required for mucous membrane attachment, once colonization occurs, numerous secreted exotoxins, including the pyrogenic toxin superantigens and exfoliative toxins, definitively cause serious human disease. Other secreted exotoxins, such as the four hemolysins (a, β, γ and δ) and Panton-Valentine leukocidin have also been suggested to contribute to significant illnesses. *S. epidermidis* does not possess the array of extracellular toxins that *S*. *aureus* does, and its primary virulence factor is considered to be its ability to form biofilms.

Since its first identification in the early 1960s (Jevons, "Celbenin" resistant staphylococci, 1961), methicillin-resistant *Staphylococcus aureus* (MRSA - also referred to as multi-resistant *Staphylococcus aureus*; or ORSA - oxacillin-resistant *Staphylococcus aureus*) has become one of the most significant nosocomial pathogens throughout the world and it is capable of causing a wide range of hospital infections. It continues to spread through new communities wherever the methods and institutions of modem medical practice are adopted, while it regularly causes epidemics in places where it has been endemic for a decade or more.

*Staphylococcus aureus* has long been recognised as one of the major human pathogens responsible for a wide range of afflictions from minor infections of the skin to wound infections, bacteraemia, infections of the central nervous system, respiratory and urinary tracts, and infections associated with intravascular devices and foreign bodies. *Staphylococcus aureus* infections can be lethal. Most *Staphylococcus aureus* strains are opportunistic pathogens that can colonize individuals, without symptoms, for either short or extended periods of time, causing disease when the immune system becomes compromised. The immense genetic repertoire of this bacterium for adapting to rapidly changing and uniformly hostile environments was repeatedly shown by the emergence of *Staphylococcus aureus* strains that acquired resistance mechanisms to virtually all antimicrobial agents shortly after the introduction of these drugs into clinical practice. A study has shown that 97% of the *Staphylococcus aureus* isolates recovered carried the resistant trait to penicillin (Sa-Leao et al., Microb. Drug. Res. 7: 237-245, 2001). The introduction of methicillin in clinical practice in 1960 was followed by the appearance of the first blood stream isolate of *Staphylococcus aureus* that was resistant not only to penicillin, streptomycin, and tetracycline (and occasionally to erythromycin), but to methicillin as well. Since the 1960s, MRSA strains have spread among hospitals isolates in several waves, which eventually disseminated these strains worldwide.

The genus can be divided into two groups: *Staphylococcus aureus* which is a pathogen of humans and the group of coagulase-negative staphylococci (CNS) which are usually part of the physiological skin flora. As part of the physiological flora of the skin and mucous membranes of humans and animals *S. epidermidis, S. hominis, S. saprophyticus and S*. *haemolyticus* are usually the predominant species in humans. Whereas CNS were previously generally considered to be non-pathogenic, recent clinical experience has shown that these organisms can indeed trigger infectious processes. They are among the most frequent sepsis pathogens particularly in immunodeficient patients as well as in premature infants and neonates. *S*. *epidermidis* dominates in infections associated with implanted foreign bodies and intravasal catheters due to its ability to adhere irreversibly to plastic surfaces. Nevertheless a positive test for CNS in clinical specimens must be judged critically since in most cases it is due to exogenous or endogenous contamination. *Staphylococcus aureus* is the most potent pathogen of the genus and causes infections as well as toxin-mediated diseases. It is part of the normal skin flora in healthy persons and is mainly found in the anterior nasal sinus, in the throat, the openings of the mammary glands and on the skin (armpit, perineal region and neck). However, it can cause severe infections when the general condition of the patient is weakened and after tissue injury, surgical interventions etc. *S*. *aureus is* described as worldwide the most frequent cause of sepsis, skin and soft-tissue infections, and pneumonia. Worldwide, an estimated 2 billion people carry some form of *S. aureus*, of which up to 53 million are thought to carry MRSA. In the US, an estimated 2.5 million individuals carry an MRSA strain (Graham et al., Ann. Intern. Med., 144 (5): 318-25, 2006).

Infections caused by MRSA are difficult to treat and show an increased mortality rate. It has been shown that patients in the US with *S. aureus* infection had, on average, a three-fold length of hospital stay, and experienced five-fold risk of in-hospital death (Noskin et al., Arch. Intern. Med. 165: 1756-1761, 2005). A study led by the CDC (published October 17, 2007) estimated that MRSA could be attributed to 94,360 serious infections and associated with 18,650 hospital stay-related deaths in the United States in 2005. Hospital Acquired MRSA (HA-MRSA) strains often are only susceptible to vancomycin. Newer drugs, such as linezolid (belonging to the newer oxazolidinones class), may also still be effective. Several newly discovered strains of MRSA show antibiotic resistance even to vancomycin and teicoplanin, and are therefore termed vancomycin intermediate-resistant *Staphylococcus aureus* (VISA) (Sieradzki et al., J. Bacteriol. 179 (8): 2557-66, 1997; Schito, Clin. Microbiol. Infect., 12 Suppl 1: 3-8, 2006). Linezolid, quinupristin/dalfopristin, daptomycin, and tigecycline are used to treat more severe infections that do not respond to glycopeptides such as vancomycin (Mongkolrattanothai et al., Clin. Infect. Dis. 37 (8): 1050-8, 2003).

In healthcare environments, MRSA can survive on surfaces and fabrics, including privacy curtains or garments worn by care providers, therefore necessitating complete surface sanitation to eliminate MRSA in areas where patients are recovering from invasive procedures, in addition to testing patients for MRSA upon admission, isolating MRSA positive patients, decolonization of MRSA positive patients, and terminal cleaning of patients rooms and all other clinical areas they occupy.

Biofilms are generally defined as an association of microorganisms growing attached to a surface and producing a slime layer of extracellular polymers in which the microbial consortium is embedded in a protective environment (for a review see: Costerton et al., Ann. Rev. Microbiol. 49: 711-45, 1995). Biofilms represent a severe problem as bacteria integrated in such a polymer matrix develop resistance to conventional antimicrobial agents. *P*. *aeruginosa* cells, for example, growing in an alginate slime matrix have been demonstrated to be resistant to antibiotics (e.g. aminoglycosides, lactam antibiotics, fluoroquinolones) and disinfectants (Govan & Deretic, Microbiol. Rev. 60: 539-74, 1996). Several mechanisms for biofilm-mediated resistance development have been proposed (Costerton et al., Science 284: 1318-22, 1999). In most natural, clinical and industrial settings bacteria are predominantly found in biofilms. Drinking water pipes, teeth or medical devices represent typical surfaces colonized by bacteria. On the one hand biofilms decrease the life time of materials through corrosive action in the industrial field, a process also referred to as "biofouling". Furthermore, microbial biofilms growing, for example, on ship hulls increase fuel consumption through increased frictional resistance and simultaneously reduce maneuverability.

Two thirds of all bacterial infections in humans are associated with biofilms (Lewis, Antimicrob. Agents Chemother. 45: 999-1007, 2001). *Pseudomonas aeruginosa*, for example, forms infectious biofilms on surfaces as diverse as cystic fibrosis lung tissue, contact lenses, and catheter tubes (Stickler et al., Appl. Environm. Microbiol. 64: 3486-90, 1998). *Burkholderia cepacia* also forms biofilms in lungs of cystic fibrosis patients and is a major industrial contaminant (Govan et al., J. Med. Microbiol. 45: 395-407, 1996). Since biofilm formation of both organisms is demonstrated to require an HSL signaling system, inhibition of their quorum sensing systems would result in an impaired ability to form biofilms and therefore in an increased susceptibility to antibacterial treatment.

Beside the role of HSL derivatives as signaling molecules of bacterial cell-to-coll communication it has been demonstrated that HSL interfere also with higher organisms. Since HSL derivatives inhibit murine and human leukocyte proliferation and TNF-alpha secretion by lipopolysaccharide (LPS) stimulated human leucocytes (Chhabra et al., J. Med. Chem. 46: 97-104, 2003) the suitability of these compounds for immunological diseases, particularly autoimmune diseases such as psoriasis, rheumatoid arthritis, multiple sclerosis and type 1(autoimmune) diabetes is indicated (WO 03/004017, WO 03/022828). Furthermore, certain HSL molecules are capable of reducing the heart beat without substantially reducing arterial blood pressure. These compounds and analogs of them could, therefore, be suitable for the treatment of cardiac tachyarrhythmia, ischemic heart disease and congestive heart failure (WO 01/26650). Additionally, HSL compounds have been reported as possible anti-allergic drug (WO 95/01175) and for the treatment of a range of diseases including cancer, breast cancer, obesity, lipid metabolism disorders, immune disease, immune deficiency or immune disorders by modulating STAT activity (WO 03/026641).

The discovery that a wide spectrum of bacterial organisms uses quorum sensing to control virulence factor production and other phenotypes such as biofilm formation makes it an attractive target for antimicrobial therapy. Pathogenic organisms using this signaling system to control virulence could potentially be rendered a virulent by blocking this cell-cell communication system. In contrast to traditional antibiotics, the risk of resistance development seems to be very low, since quorum sensing blocking agents would not kill the organism but disturb signal transduction pathways. The are only few non-HSL-based antimicrobials described in the literature which are supposed to interfere specifically with HSL-regulated processes, for example halogenated furanone derivatives which are structurally similar to HSLs and have been isolated from red marine algae *Delisea pulchra* (WO 96/29392; Hentzer et al., Microbiology 148:87-102, 2002). However, the use of most of these furanone compounds is limited due to their toxicity making them unsuitable for veterinary and medical applications. Furthermore, Smith et al. (Chem. Biol. 10:81-9, 2003; Chem. Biol. 10: 563-71,2003) published *Pseudomonas aeruginosa* HSL analogs with slight structural variations targeted to the HSL moiety which act both as quorum sensing agonists and antagonists. Recently, quorum sensing inhibitor RIP has been demonstrated to reduce *Staphylococcus aureus* biofilm infections in rats (Balaban et al., Antimicrobial Agents Chemotherapy 51, 6: 2226-9,2007).

It is an object of the present invention to provide compounds blocking specifically quorum sensing regulated processes without inhibiting bacterial growth. Accordingly, we have been able to find compounds that can significantly inhibit quorum sensing dependant biofilm formation of several bacteria, bacterial pathogens, more preferably selected from the group *Pseudomonas aeruginosa, Staphylococcus aureus* and *Staphylococcus epidermidis,* most preferably MRSA strains. In contrast to the furanones, the compounds of this invention do not exhibit any toxic effect and are, therefore, suitable for applications in a wide area. Such applications could be the use of the compounds for instance as new antibacterial therapeutics, disinfectants, antifouling coatings or additives to medical devices. In contrast to traditional antibacterial agents, the compounds of the present invention do not kill microorganisms, but render them avirulent. The advantages of this alternative strategy are that the emergence of bacterial resistance against such antimicrobials is extremely improbable, and that the bacterial population becomes more susceptible to the host immune-response or to a treatment with conventional antibacterial agents. The combination of the compounds of this invention with conventional antibiotics or biocides are supposed to exhibit synergistic effects which are beneficial especially for prophylaxis and treatment of bacteria, in particular multi-resistant strains of *Pseudomonas aeruginosa* or *Staphylococcus aureus*. Thus, in one aspect, the invention refers to a method for inhibiting the formation of bacterial biofilms by exposing the bacteria to a new class of compounds with an inhibitory effect on bacterial signaling.

### Detailed description of the embodiments

The present invention is directed to compounds of the general formula (I) and pharmaceutically acceptable salts and solvates thereof, 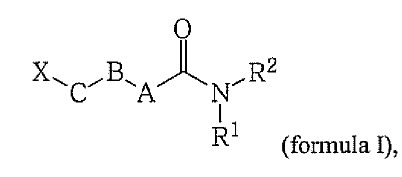 wherein
A is CH₂ or NR¹¹;
B is CH₂, CO, or O;
C is CH₂ or O;
- X is: C₂-C₂₀ alkyl, optionally substituted with one or more R^{alk};
- R^{alk} is: independently alkyl or alkyl-OR, wherein R may be H or part of an ester group
wherein the acid part comprises 1-6 carbon atoms.
- R¹ is: H, alkyl, cycloalkyl, heterocycloalkyl, aryl or heteroaryl;
- R² is: alkyl, cycloalkyl, heterocycloalkyl, aryl, heteroaryl or 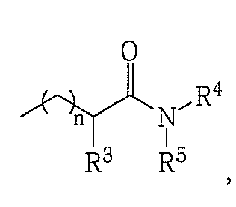
whereby n is an integer from 1 to 3;
- R³ is: H, alkyl or R¹ and R³ taken together may form a 5- to 7-membered heterocyclic ring, which may optionally contain one or more double bonds and which may optionally contain one or more additional heteroatoms;
- R⁴ is: H, alkyl, cycloalkyl, heterocycloalkyl, aryl or heteroaryl;
- R⁵ is: H, alkyl, cycloalkyl, heterocycloalkyl, aryl, heteroaryl or R⁴ and R⁵ taken together may form a 5- to 7-membered heterocyclic ring, which may optionally contain one or more double bonds and which may optionally contain one or more additional heteroatoms and which heterocyclic ring may also be part of a ring system which may optionally be an aromatic ring system;
- Rⁿ is: alkyl, cycloalkyl, heterocycloalkyl, aryl, heteroaryl, OH, O-alkyl
- n is: an integer from 1 to 3;
with the proviso that
if R² is an optionally substituted 1-methyl-pyrazol-5-yl, then at least one of A,
B or C is other than CH₂;
if B is CO, then at least one of A or C must be other than CH₂.

A preferred embodiment are compounds of the general formula (I) and pharmaceutically acceptable salts and solvates thereof, 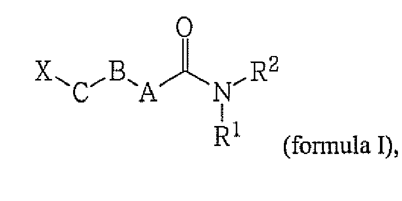 wherein 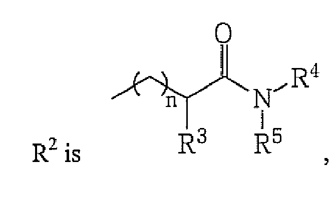 whereby n is an integer from 1 to 3; and
wherein the further groups are as defined above.

Another preferred embodiment are compounds of the general formula (I) and pharmaceutically acceptable salts and solvates thereof, 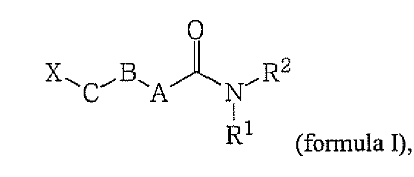 wherein 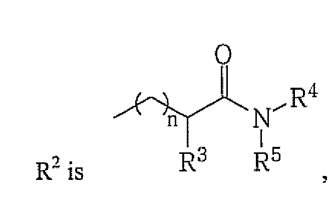 whereby n is an integer from 1 to 3;
R¹ and R³ taken together form a 5- to 7-membered heterocyclic ring, which may optionally contain one or more double bonds and which may optionally contain one or more additional heteroatoms; and
wherein the further groups are as defined above.

Another preferred embodiment are compounds of the general formula (I) and pharmaceutically acceptable salts and solvates thereof, 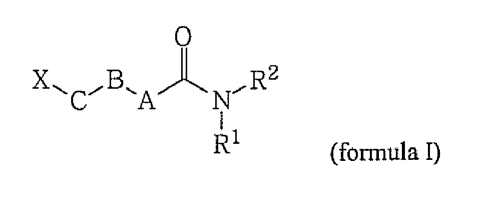 wherein
A is CH₂; B is O; C is CH₂;
or A is CH₂; B is CH₂ and C is O;
or A is NRⁿ; B is CO and C is CH₂;
or A is NRⁿ; B is CH₂ and C is CH₂;
or A is CH₂; B is CH₂ and C is CH₂;
R² is cycloalkyl, heterocycloalkyl, aryl or heteroaryl, more preferably heteroaryl, optionally substituted with a group selected from halogen, alkyl, cycloalkyl, heterocycloalkyl, aryl or heteroaryl, even more preferably pyrazolyl, optionally substituted a group selected from halogen, alkyl, cycloalkyl, heterocycloalkyl, aryl or heteroaryl; and
wherein the further groups are as defined above;
with the proviso that if
if R² is an optionally substituted 1-methyl-pyrazol-5-yl, then at least one of A, B or C is other than CH₂.

Another preferred embodiment are compounds according to claim 1, wherein X is C₆-C₁₀ alkyl, optionally substituted with one or more R^{alk} as defined above; and wherein the further groups are as defined in claim 1.

A particularly preferred embodiment of the present invention is a compound selected from the following formulae II to X;

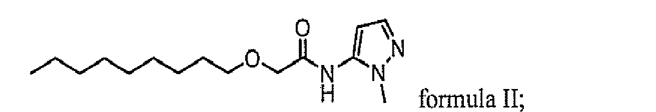

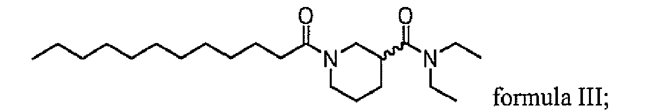

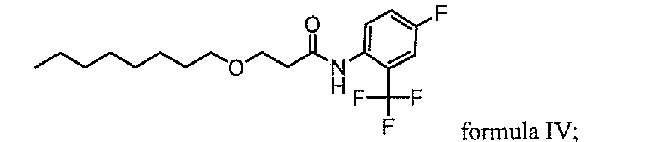

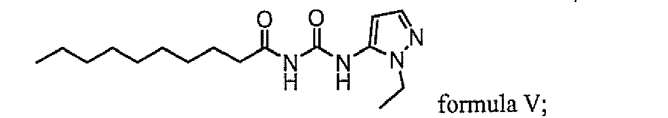

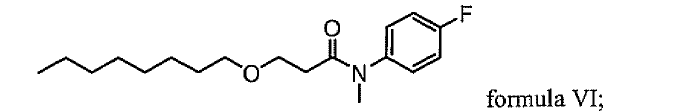

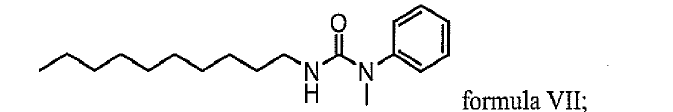

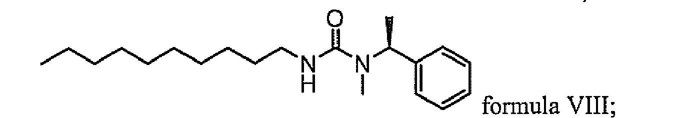

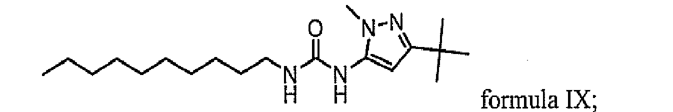

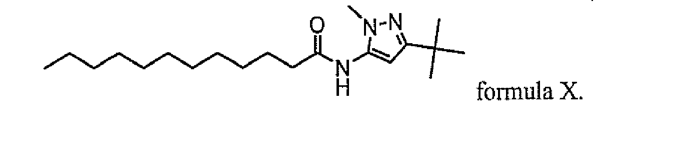

Another embodiment is a compound according to the present invention as a medicament or antibacterial agent.

Another embodiment is a compound according to the present invention as a medicament or antibacterial agent for the treatment or prevention of bacterial diseases or damages.

A preferred embodiment is a compound according to the present invention as a medicament or antibacterial agent for the treatment or prevention of bacterial diseases or damages caused by gram-positive bacteria.

A more preferred embodiment is a compound according to the present invention as a medicament or antibacterial agent for the treatment or prevention of bacterial diseases caused by a *Staphylococcus* strain.

A more preferred embodiment is a compound according to the present invention as a medicament or antibacterial agent for the treatment or prevention of bacterial diseases caused by MRSA bacteria.

An even more preferred embodiment is a compound according to the present invention as a medicament or antibacterial agent for the treatment or prevention of bacterial diseases caused by bacteria selected from the group *Pseudomonas aeruginosa, Staphylococcus aureus* (MRSA) and *Staphylococcus epidermidis*.

A further embodiment is the use of a compound according to the present invention as an antifouling agent.

In the context of the above embodiments, prevention of a disease can also be effected by treatment of surfaces or matter that is contaminated with one or more of said bacterial strains. Means for such treatment are explained herein in further detail.

Another embodiment is a compound according to the present invention for the treatment of biofilms or for inhibiting biofilm formation during a bacterial infection in a patient. A patient may be a human or non-human patient.

Another preferred embodiment is a compound according to the present invention for the treatment or prevention of diseases selected from sepsis, endocarditis, respiratory and pulmonary infections (preferably in immunocompromized and cystic fibrosis patients), bacteremia, central nervous system infections, ear infections including external otitis, eye infections, bone and joint infections, urinary tract infections, gastrointestinal infections and skin and soft tissue infections including wound infections, pyoderma and dermatitis.

Another embodiment is the use of a compound according to the present invention as an antibacterial agent for the prevention and/or inhibition of biofilm growth on surfaces.

Another preferred embodiment is the use of a compound according to the present invention as an antibacterial agent for the prevention and/or inhibition of biofilm growth on medical appliances.

Another embodiment is a method for treating or preventing a disease comprising administering to a patient in need thereof a compound according to the present invention.

Another preferred embodiment is a method for treating or preventing a bacterial disease comprising administering to a patient in need thereof a compound according to the present invention.

Another preferred embodiment is a method for treating or preventing bacterial diseases or damages caused by gram-positive bacteria comprising administering to a patient in need thereof a compound according to the present invention.

Another even more preferred embodiment is a method for treating or preventing bacterial diseases caused by a *Staphyllococcus* strain comprising administering to a patient in need thereof a compound according to the present invention.

Another even more preferred embodiment is a method for treating or preventing bacterial diseases caused by a MRSA bacteria comprising administering to a patient in need thereof a compound according to the present invention.

Another even more preferred embodiment is a method for treating or preventing bacterial diseases caused by bacteria selected from the group *Pseudomonas aeruginosa*, *Staphylococcus aureus* (MRSA) and *Staphylococcus epidermidis* comprising administering to a patient in need thereof a compound according to the present invention.

Another preferred embodiment is a method for treating or preventing diseases selected from food-borne infections, endocarditis, respiratory and pulmonary infections (preferably in immunocompromized and cystic fibrosis patients), bacteremia, central nervous system infections, ear infections including external otitis, eye infections, bone and joint infections, urinary tract infections, gastrointestinal infections and skin and soft tissue infections including wound infections, pyoderma and dermatitis.

Another preferred embodiment is a method for treating or preventing biofilms or for inhibiting biofilm formation during a bacterial infection in a patient caused by MRSA bacteria comprising administering to a patient in need thereof a compound according to the present invention.

Another embodiment is the use of a compound according to the present invention as an antibacterial agent.

Another preferred embodiment is the use of a compound according to the present invention as an antibacterial agent for the prevention and/or inhibition of biofilm growth on surfaces.

Another preferred embodiment is the use of a compound according to the present invention as an antibacterial agent for the prevention and/or inhibition of biofilm growth on medical appliances.

Another embodiment is the use of a compound according to the present invention as an antifouling agent.

An alkyl group, if not stated otherwise, denotes a linear or branched C₁-C₆-alkyl, preferably a linear or branched chain of one to five carbon atoms, a linear or branched C₂-C₆-alkenyl or a linear or branched C₂-C₆-alkinyl group, which can optionally be substituted by one or more substituents R^{X}, preferably, the C₁-C₆-alkyl, C₂-C₆-alkenyl and C₂-C₆-alklnyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R^{X})₃, -C₂(R^{X})₅, -CH₂-C(R^{X})₃, -C₃(R^{X})₇, -C₂H₄-C(R^{X})₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH-C(CH₃)₂, -C(CH₃)=C(CH₃)₂, -C₃H₆-C≡CH, -O≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH-CH-C≡C-CH₃, -C=C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)-CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅.

A halogen group is chlorine, bromine, fluorine or iodine.

A cycloalkyl group denotes a cyclic 3- to 8-membered non-aromatic carbocyclic ring which may contain one or more double bonds and which optionally is substituted by one or more R^{X} as defined below. For example, this group can be selected from cyclopropyl, cyclobutyl, cyclopentyl, and cyclohexyl. Such a cycloalkyl group may also be substituted with one or more groups independently selected from -ORⁿ, SRⁿ, -N(R¹¹)₂, =O, =S or =NRⁿ.

A heterocycloalkyl group denotes a cyclic 3- to 8-membered non-aromatic ring which contains at least one heteroatomic group independently selected from O, S, N, NRⁿ, SO, SO₂, which may contain one or more double bonds and which optionally is substituted by one or more R^{X} as defined below. For example, this group can be selected from 1-azetidinyl, 2-azetidinyl, 3-azetidinyl, 1-pyrrolidinyl, 2-pyrrolidinyl, 3-pyrrolidinyl, 1-piperidinyl, 2-piperidinyl, 3-piperidinyl, and 4-piperidinyl, dihydropyrrolyl, dihydrofuryl, dihydrothienyl, dihydroimidazolyl, dihydrooxazolyl, dihydroisoxazolyl, dihydrothiazolyl, and dihydroisothiazolyl. Such a heterocycloalkyl group may also be substituted with one or more groups independently selected from -ORⁿ, SRⁿ, -N(Rⁿ)₂, =O, =S or =NRⁿ.

An aryl group preferably denotes an aromatic group having six to fifteen carbon atoms, which can optionally be substituted by one or more substituents R^{X}, where R^{X} is as defined below and may also be substituted by one or more -ORⁿ, SRⁿ, -N(Rⁿ)₂; the aryl group is preferably a phenyl group, a benzyl group, -C₂H₄-Ph, -CH=CH-Ph, -C≡C-Ph, -o-C₆H₄-R^{X}, -m-C₆H₄-R^{X}, -p-C₆H₄-R^{X}, -o-CH₂-C₆H₄-R^{X}, -m-CH₂-C₆H₄-R^{X}, -p-CH₂-C₆H₄-R^{X}, 1-naphthyl, 2-naphthyl, 1-anthracenyl or 2-anthracenyl.

A heteroaryl group denotes a 5-or 6-membered heterocyclic aromatic group which contains at least one heteroatom selected from O, N, S. This heterocyclic group can be fused to another, optionally aromatic ring. For example, this group can be selected from an oxazol-2-yl, thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1,2,5-thiadiazol-4-yl, 1-imidazolyl, 2-imidazolyl, 4-imidazolyl, 1-pyrrolyl, 2-pyrrolyl, 3-pyrrolyl, 2-furyl, 3- furyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, isoxazol-3-yl, isoxazol-4-yl, isoxazol-5-yl, quinolinyl, tetrahydroquinolinyl, isoquinolinyl, tetraliydroisoquinolinyl group. This heterocyclic group can optionally be substituted by one or more substituents R^{X}, where R^{X} is as defined below and may also be substituted by one or more -ORⁿ, SRⁿ, -N(Rⁿ)₂,

R^{X} is independently H, -CO₂R^{Y}, -CONHR^{Y}, -CR^{Y}O, -SO2NR^{Y}, -NR^{Y}-CO-haloalkyl, NO₂, NR^{Y}-SO₂-haloalkyl, -NR^{Y}-SO₂-alkyl, -SO₂-alkyl, NR^{Y}-CO-alkyl, -CN, alkyl, cycloalkyl, alkylamino, alkoxy, -OH, -SH, alkylthio, hydroxyalkyl, hydroxyalkylamino, halogen, haloalkyl, haloalkyloxy, aryl, or heteroaryl.

R^{Y} is independently H, haloalkyl, hydroxyalkyl, alkyl, cycloalkyl, aryl, or heteroaryl.

RX and RY hereby may not be substituted by a second or otherwise further residue selected fom RX and/or RY. This is to be understood such that oligomeric or polymeric residues comprised of RX and/or RY units are not within the scope of the present invention.

Examples of pharmaceutically acceptable salts comprise without limitation non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfate derived from p-toluene- sulfonic acid and others. Such salts can be produced by methods known to someone of skill in the art and described in the prior art.

Other salts like oxalate derived from oxalic acid, which is not considered as pharmaceutically acceptable can be appropriate as intermediates for the production of compounds of the Formula(I) or a pharmaceutically acceptable salt thereof or stereoisomer thereof.

In general, the compounds of the present invention can be used to inhibit biofilm formation of bacteria employing quorum sensing signaling systems. Preferably, the compounds can be applied to Gram-positive and Gram-negative bacteria, preferably to *Staphylococcus* strains, and more preferably to *Pseudomonas aeruginosa*, *Staphylococcus aureus* including MRSA strains and *Staphylococcus epidermidis.* In the following it is explained that the compounds of the present invention can be used as antibacterial agents in various applications.

In a preferred form, the compounds of Formula(I) are useful for the treatment of a variety of human, animal and plant diseases, where bacterial pathogens regulate the expression of virulence genes and other phenotypes, e.g. biofilm formation, through quorum sensing.

The compounds according to the present invention are useful for the treatment of mammalian, in particular human, diseases caused by bacteria through the inhibition of the bacterial quorum sensing cascade avoiding biofilms formation and rendering the pathogen avirulent. Such diseases include sepsis, endocarditis, respiratory and pulmonary infections (preferably in immunocompromized and cystic fibrosis patients), bacteremia, central nervous system infections, ear infections including external otitis, eye infections, bone and joint infections, urinary tract infections, gastrointestinal infections and skin and soft tissue infections including wound infections, pyoderma and dermatitis. According to the present standard of knowledge, in all of these diseases bacteria of the genus *Pseudomonas* and/or *Staphylococcus* are involved.

Furthermore, the compounds can be used for the treatment of pulmonary infections caused by *Burkholderia cepacia* (preferably in immunocompromized and cystic fibrosis patients), gastroenteritis and wound infections caused by *Aeromonas hydrophila*, sepsis in tropical and subtropical areas caused by *Chromobacterium violaceum*, diarrhea with blood and hemolytic uremic syndrome (HUS) caused by Escherichia coli, yersiniosis triggered by *Yersinia enterocolitica* and *Y*. *pseudotuberculosis*, transfusion-related sepsis and fistulous pyoderma caused by *Serratia liquefaciens*. Another area where compounds according to the present invention can preferably be used is the treatment of prevention of food-borne infections caused by the genera *Salmonella* and/or *Escherichia* and/or *Listeria*.

The compounds can be used in the treatment of immunological diseases, particularly autoimmune diseases such as psoriasis, rheumatoid arthritis, multiple sclerosis and type 1 (autoimmune) diabetes, of cardiovascular diseases such as cardiac tachyarrhythmia, ischemic heart disease, congestive heart failure, of allergic diseases and of diseases including cancer, breast cancer, obesity, lipid metabolism disorders, immune disease, immune deficiency or immune disorders.

The compounds can be used to prevent and/or treat plant diseases, where inhibition of the HSL-mediated signaling system reduces or abolishes virulence of bacterial plant pathogens. Such diseases include crown gall tumors caused by *Agrobacteriuna tumefaciens*, soft rot caused by *Burkholderia cepacia, Erwinia carotovora* and *Erwinia chrysanthemi*, sweet corn and maize infections caused by *Pantoea stewartii* and wilt disease caused by *Ralstonia solanacearum*.

In a fourth embodiment, the compounds can be used for the prevention and/or treatment of animal diseases, preferably fish diseases such as septicemia caused by *Aeromonas hydrophila* and *Vibroanguillarum furunculosis* in salmonids caused by *Aeromonas salmonicida*, prawn infections caused by *Vibrio harveyi* and enteric redmouth disease caused by *Yersinia ruckeri,* but also for the prevention and/or treatment of insect diseases caused, for example, by *Xenorhabdus nematophilus*.

In general, the present invention provides a method for reducing the virulence of bacterial pathogens by inhibiting biofilm formation. In a preferred form, a method is provided to remove, diminish, detach or disperse a bacterial biofilm from a living or nonliving surface by treating the surface with a compound of Formula(I). This method is also useful to prevent biofilm formation on a living or nonliving surface by treating the surface with a compound of Formula (I) before bacterial colonization can initialize. The term "biofilm" refers to cell aggregations comprising either a single type of organism or a mixture of more than one organism, then also referred to as "mixed biofilms". It is clear to persons skilled in the art, that the compounds of the present invention can be applied in a wide variety of different fields such as environmental, industrial and medical applications in order to prevent and/or treat damages or diseases caused by bacteria.

In one aspect, the compounds of Formula (I) can be used for all kinds of surfaces in private and public areas, where it is beneficial to inhibit colonization of Gram-positive and Gram-negative bacteria, preferably MRSA strains, preferably of *Staphylococcus* strains. The compounds here can be used in form of a solution, powder or as a coating. The compound is preferably applied to the surface as a solution of the compound, alone or together with other materials such as conventional surfactants, preferably sodium dodecyl sulfate, or detergents, biocides, fungicides, antibiotics, pH regulators, perfumes, dyes or colorants. In combination with a bacteriocidal agent, e.g. the compounds of Formula (I) inhibit virulence or biofilm formation whilst the bacteriocidal agent kills the pathogens, and therefore exhibits a synergistic effect.

In one embodiment, the compounds can be used as antibacterial agent for topical use in cleaning and treatment solutions such as disinfectants, detergents, household cleaner and washing powder formulations in the form of a spray or a dispensable liquid. In a preferred form, these solutions can be applied to windows, floors, clothes, kitchen and bathroom surfaces and other surfaces in the area of food preparation and personal hygiene.

In addition, the compounds of Formula (I) can be used as antibacterial ingredients in personal hygiene articles, toiletries and cosmetics such as dentifrices, mouthwashes, soaps, shampoos, shower gels, ointments, creams, lotions, deodorants and disinfectants and storage solutions for contact lenses. In the case of contact lenses the compounds of Formula (I) can also be applied as coating or additive to the lens material.

In another embodiment, the compounds can be used to prevent or treat bacterial biofilms in industrial settings such as ship hulls, paper and metal manufacturing, oil recovery, food processing and other applications where process disturbances are referred to biofouling on surfaces. The compounds here can be used in form of a solution, paint or coating, for example as an ingredient in cooling lubricants. The compounds can also be applied to water processing plants or drinking water distribution systems where the colonized surface (preferably by *Pseudomonas aeruginosa*) is preferably the inside of an aqueous liquid system such as water pipes, water injection jets, heat exchangers and cooling towers. Until now biocides are the preferred tools to encounter these problems, but since biocides do not have a high specificity for bacteria, they are often toxic to humans as well. This can be circumvented by the application of the compounds of the present invention.

In a further embodiment, the present invention relates to a method of inhibiting and/or preventing medical device-associated bacterial infections. The invention provides articles coated, incorporated and/or impregnated with a compound of Formula(I) in order to inhibit and/or prevent biofilm formation thereon. The articles are preferably surgical instruments, blood bag systems or medical devices; more preferably either permanently implanted devices such as artificial heart valve, prostethic joint, voice prosthesis, stent, shunt or not permanently implanted devices such as endotracheal or gastrointestinal tube, pacemaker, surgical pin or indwelling catheter.

In a more preferred form, the indwelling catheters are urinary catheters, vascular catheters, peritoneal dialysis catheter, central venous catheters and needledess connectors.

The catheter materials can be polyvinylchloride, polyethylene, latex, teflon or similar polymeric materials, but preferably polyurethane and silicone or a mixture thereof. In order to reduce the risk of catheter-related bacterial infections, several catheters coated and/or impregnated with antiseptic or antimicrobial agents such as chlorhexidine/silver-sulfadiazine andminocycline/rifampin, respectively, have been developed. Furthermore, collection bags or layers sandwiched between an external surface sheath and a luminal silicone sheath have been constructed to overcome rapid loss of antimicrobial activity.

Nevertheless, the emerging risk of bacterial resistance against traditional antibiotics limits the routine use of antibiotic-coated catheters. The compounds of the present invention, however, offer the possibility to effectively reduce catheter-related bacterial infections with a low risk of resistance development due to a novel therapeutic strategy targeting highly sensitive signal transduction mechanisms in bacteria. The preferred form of application is the coating and/or impregnating of catheter materials on both the inner and outer catheter surfaces.

More preferably, the compounds of Formula (I) can be included in a mixture of antibacterial agents released continuously from a catheter-associated depot into the environment.

In a further embodiment, the compounds of the present invention and their pharmacologically acceptable salts can be administered directly to animals, preferably to mammals, and in particular to humans as antibiotics per se, as mixtures with one another or in the form of pharmaceutical preparations which allow enteral or parenteral use and which as active constituent contain an effective dose of at least one compound of the Formula (I) or a salt thereof, in addition to customary pharmaceutical excipients and additives. The compounds of Formula (I) can also be administered in form of their salts, which are obtainable by reacting the respective compounds with physiologically acceptable acids and bases.

The therapeutics can be administered orally, e.g., in the form of pills, tablets, coated tablets, sugar coated tablets, lozenges, hard and soft gelatin capsules, solutions, syrups, emulsions or suspensions or as aerosol mixtures. Administration, however, can also be carried out rectally, e.g. in the form of suppositories, or parenterally, e.g. in the form of injections or infusions, or percutaneously, e.g. in the form of ointments, creams or tinctures.

In addition to the active compounds of Formula (I) the pharmaceutical composition can contain further customary, usually inert carrier materials or excipients. Thus, the pharmaceutical preparations can also contain additives or adjuvants commonly used in galenic formulations, such as, e. g., fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for modifying the osmotic pressure, coating agents or antioxidants. They can also contain two or more compounds of the Formula(I) or their pharmacologically acceptable salts and also other therapeutically active substances.

Thus, the compounds of the present invention can be used alone, in combination with other compounds of this invention or in combination with other active compounds, for example with active ingredients already known for the treatment of the afore mentioned diseases, whereby in the latter case a favorable additive effect is noticed. Suitable amounts to be administered to mammalian in particular humans range from 5 to 1000 mg.

To prepare the pharmaceutical preparations, pharmaceutically inert inorganic or organic excipients can be used. To prepare pills, tablets, coated tablets and hard gelatin capsules, e.g. lactose, corn starch or derivatives thereof, talc, stearic acid or its salts, etc. can be used. Excipients for soft gelatin capsules and suppositories are, e.g. fats, waxes, semi-solid and liquid polyols, natural or hardened oils etc. Suitable excipients for the production of solutions and syrups are, e.g. water, alcohol, sucrose, invert sugar, glucose, polyols etc. Suitable excipients for the production of injection solutions are, e.g. water, alcohol, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 0.0 to 100 mg/kg animal body weight preferably 1 to 50 mg/kg. Suitable dosage rates for larger mammals, e.g. humans, are of the order of from about 10 mg/day to 3 g/day, conveniently administered once, in divided doses 2 to 4 times a day, or in sustained release form.

In general, a daily dose of approximately 0.1 mg to 5000 mg, preferably 10 to 500 mg, per mammalian in particular human individual is appropriate in the case of the oral administration which is the preferred form of administration according to the invention. In the case of other administration forms too, the daily dose is in similar ranges. The compounds of Formula (I) can also be used in the form of a precursor (prodrug) or a suitably modified form that releases the active compound *in vivo*.

In a further embodiment, the compounds of the present invention can be used as pharmacologically active components or ingredients of medical devices, instruments and articles with an effective dose of at least one compound of the Formula (I) or a salt thereof. The amount of the compounds used to coat for example medical device surfaces varies to some extent with the coating method and the application field. In general, however, the concentration ranges from about 0.01 mg/cm² to about 100 mg/cm². In a similar way the amount of the compounds has to be adjusted to the application mode if the compounds of the invention are used as components or ingredients in cleaning or treatment solutions. In general, effective dosages range from about 0.1 µM to about 1000 mM.

### EXAMPLES

### TEST METHOD: MBEC^{™} Assay

The compound to be tested was dissolved in DMSO to obtain a concentration that was 100 times that of the final concentration required (1mM for the 10µM assay and 10mM for the 100µM assay). 1.5 µL of the above solution was placed into each well of a 96 well plate (final volume in each well = 150 µL, final DMSO concentration of 1% (v/v)).

To grow the organism and form a biofilm, a cryogenic stock of the test organism (at -70°C) was used. A first sub-culture was streaked out onto TSA (tryptic soy agar), upon which the plate was incubated at 35±2°C for 24 hours and stored wrapped in parafilm at 4 °C afterwards. From this first sub-culture, a second sub-culture was streaked out onto TSA. The plate was incubated at 35±2°C for 24 hours. The second sub-culture was used within 24 hours starting from the time it was first removed from incubation. Using the second sub-culture an inoculum in 3 mL sterile water that matches a 0.5 McFarland Standard (1.5 x 10⁸ cells per mL) was created in a glass test tube using a sterile cotton swab. 2.2 mL of this solution was diluted in 22mL 100% TSB (tryptic soy broth) medium to a cell density of approximately 6.0 x 10⁵ cells per mL. The diluted organism was inverted 3-5 times to achieve uniform mixing of the organism. One sample (100µl) of the diluted organism was used for an inoculum check by serially diluting and spot plating on TSA.

To prepare a challenge plate, 20 µl of the diluted organism was added to each well of a 96 well plate except the sterility control wells. The test compounds were added to the wells to make up final concentrations of 10 µM and 100 µM (1.5 µL /well) in the designated location in the test plate. 128.5 µL of 100% universal neutralizer (1% Tween 80 in Cation Adjusted Mueller Hinton Broth, CAMHB) was placed in each of the wells. 130 µL of media was added to the growth control wells. 128.5 µL of media + 1.5 µL of DMSO were added to a second set of growth control wells. 150 µL of media was added to the sterility controls. Each sample was run in triplicate. The plate was covered by a lid with a peg per well and wrapped in parafilm and placed on a shaker in a humidified incubator (GeneVac) at 35±2°C for 24 hours set at 110 rpm. Rinse plate(s) of 0.9% saline (200 µL per well) were prepared in a sterile 96 well microlitre plate. Lid-associated pegs were rinsed in 0.9% saline for approximately 1-2 minutes. The peg lid was transferred to the recovery media (1.0% Tween 80 in CAMHB) then sonicated for 30 minutes to dislodge surviving biofilm. Following sonication, 100 µl from each well of the 96 well plate was placed into the first 12 wells of the first row of a 96 well microtiter plate. 180 µl of 0.9% sterile saline was placed in the remaining rows. A serial dilution (10⁰-10⁻⁷) was prepared by moving 20 µl down each of the 8 rows. 20µl from each well was removed and spot plated on a prepared TSA. In the remaining recovery plate, 100 µl of fresh growth media (1.0% Tween 80 in CAMHB) was placed in each well to replace the amount removed. The recovery plate was covered with a regular lid and incubated at 35±2°C for 24 hours and read visually to confirm antimicrobial activity of the test compounds.

The compounds according to the present invention have been tested regarding their efficiency to inhibit biofilm formation of *Staphylococcus aureus* U of C#18 (MRSA), *Staphylococcus aureus* 456 (MRSA), *Staphylococcus epidermidis* RP62A, and *Pseudomonas aeruginosa* PAO1. The results are shown in Table 1.

**Table 1:**

| **No.** | | ***S. aureus U of C* #18(MRSA)** | ***S. aureus* 456(MRSA)** | ***S. epidermidis* RP62A** | ***P. aeruginosa* PAO1** |
|---|---|---|---|---|---|
| **1** | 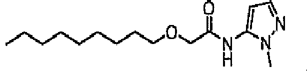 | 0.40/0.77 | 0.26/1.18 | 0.00/0.00 | 27 |
| **2** | 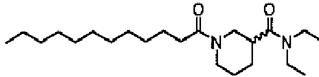 | 1.56/5.38 | 1.75/5.79 | 0.00/3.50 | 12 |
| **3** | 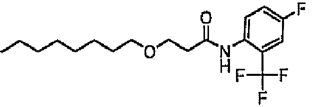 | 0.11/0.19 | 0.19/0.49 | 0.00/0.00 | 19 |
| **4** | 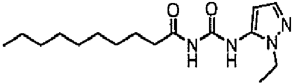 | 1.13/1.49 | 1.14/3.28 | 0.00/0.78 | 11 |
| **5** | 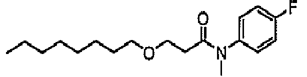 | -0.21/1.29 | 0.54/1.86 | 0.00/0.00 | 24 |
| **6** | 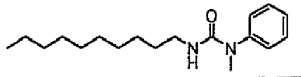 | 0.72/1.08 | 1.61/2.30 | 0.00/4.45 | 9 |
| **7** | 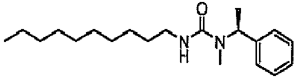 | 0.84/1.12 | 0.53/1.26 | 0.00/0.00 | 4 |
| **8** | 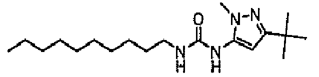 | 0.26/0.80 | 0.42/1.04 | 0.00/0.00 | 3 |
| **9** | 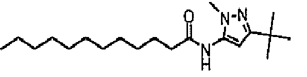 | 0.42/0.46 | 0.65/1.06 | 0.00/0.00 | 12 |

| | | | | | |
|---|---|---|---|---|---|
| Potency data are shown as inhibitory concentration IC50 [µM] for *P. aeruginosa*, and for all other strains as logR (log reduction) at two compound concentrations (10 µM / 100 µM). | | | | | |

## Claims

1. A compound of the general formula (I) and pharmaceutically acceptable salts and solvates thereof, 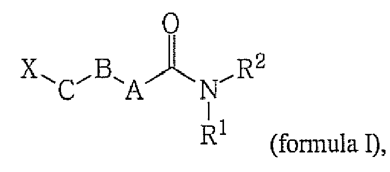 wherein
A is CH₂ or NRⁿ;
B is CH₂, CO, or O;
C is CH₂ or O;
X is C₂-C₂₀ alkyl, optionally substituted with one or more R^{alk};
R^{alk} is independently alkyl or alkyl-OR, wherein R may be H or part of an ester group wherein the acid part comprises 1-6 carbon atoms.
R¹ is H, alkyl, cycloalkyl, heterocycloalkyl, aryl or heteroaryl;
R² is alkyl, cycloalkyl, heterocycloalkyl, aryl, heteroaryl or 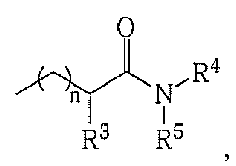 whereby n is an integer from 1 to 3;
R³ is H, alkyl or R¹ and R³ taken together may form a 5- to 7-membered heterocyclic ring, which may optionally contain one or more double bonds and which may optionally contain one or more additional heteroatoms;
R⁴ is H, alkyl, cycloalkyl, heterocycloalkyl, aryl or heteroaryl;
R⁵ is H, alkyl, cycloalkyl, heterocycloalkyl, aryl, heteroaryl or R⁴ and R⁵ taken together may form a 5- to 7-membered heterocyclic ring, which may optionally contain one or more double bonds and which may optionally contain one or more additional heteroatoms and which heterocyclic ring may also be part of a ring system which may optionally be an aromatic ring system;
Rⁿ is alkyl, cycloalkyl, heterocycloalkyl, aryl, heteroaryl, OH, O-alkyl
n is an integer from 1 to 3;
with the proviso that
if R² is an optionally substituted 1-methyl-pyrazol-5-yl, then at least one of A,
B or C is other than CH₂;
if B is CO, then at least one of A or C must be other than CH₂.

2. A compound of the general formula (I) and pharmaceutically acceptable salts and solvates thereof, 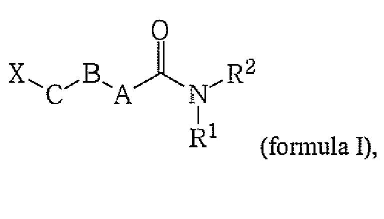 wherein 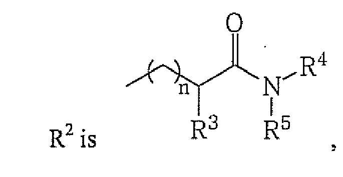 whereby n is an integer from 1 to 3; and
wherein the further groups are as defined in claim 1.

3. A compound of the general formula (I) and pharmaceutically acceptable salts and solvates thereof, 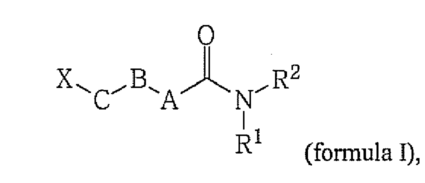 wherein 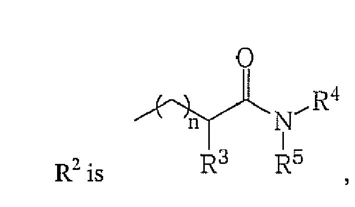 whereby n is an integer from 1 to 3;
R¹ and R³ taken together form a 5- to 7-membered heterocyclic ring, which may optionally contain one or more double bonds and which may optionally contain one or more additional heteroatoms; and
wherein the further groups are as defined in claim 1.

4. A compound of the general formula (I) and pharmaceutically acceptable salts and solvates thereof, 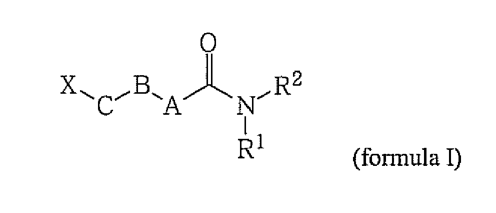 wherein
A is CH₂; B is O; C is CH₂;
or A is CH₂; B is CH₂ and C is O;
or A is NRⁿ; B is CO and C is CH₂;
or A is NRⁿ; B is CH₂ and C is CH₂;
or A is CH₂; B is CH₂ and C is CH₂;
R² is cycloalkyl, heterocycloalkyl, aryl or heteroaryl; and
wherein the further groups are as defined in claim 1;
with the proviso that if
if R² is an optionally substituted 1-methyl-pyrazol-5-yl, then at least one of A,
B or C is other than CH₂.

5. A compound according to claim 4, whereby R² is heteroaryl, optionally substituted by one or more of the following groups: Halogen, alkyl, cycloalkyl, heterocycloalkyl, aryl or heteroaryl.

6. A compound according to claim 4, whereby R² is pyrazolyl, optionally substituted by one or more of the following groups: Halogen, alkyl, cycloalkyl, heterocycloalkyl, aryl or heteroaryl.

7. A compound according to any of claims 1 to 6, wherein
X is C₆-C₁₀ alkyl, optionally substituted with one or more R^{alk} as definded in claim 1;
and
wherein the further groups are as defined in claim 1.

8. A compound according to any of claims 1 to 7 as a medicament or antibacterial agent.

9. A compound according to any of claims 1 to 7 as a medicament or antibacterial agent for the treatment or prevention of bacterial diseases or damages.

10. A compound according to any of claims 1 to 7 as a medicament or antibacterial agent for the treatment or prevention of bacterial diseases or damages caused by gram-positive bacteria.

11. A compound according to any of claims 1 to 7 as a medicament or antibacterial agent for the treatment or prevention of bacterial diseases caused by a *Staphylococcus* strain.

12. A compound according to any of claims 1 to 7 as a medicament or antibacterial agent for the treatment or prevention of bacterial diseases caused by MRSA bacteria.

13. A compound according to any of claims 1 to 7 as a medicament or antibacterial agent for the treatment or prevention of bacterial diseases caused by bacteria selected from the group *Pseudomonas aeruginosa, Staphylococcus aureus* (MRSA) and
*Staphylococcus epidermidis.*

14. A compound according to any of claims 1 to 7 for the treatment or prevention of diseases selected from of sepsis, endocarditis, respiratory and pulmonary infections (preferably in immunocompromized and cystic fibrosis patients), bacteremia, central nervous system infections, ear infections including external otitis, eye infections, bone and joint infections, urinary tract infections, gastrointestinal infections and skin and soft tissue infections including wound infections, pyoderma and dermatitis.

15. A compound according to any of claims 1 to 7 for the treatment of biofilms or for inhibiting biofilm formation during a bacterial infection in a patient.

16. The use of a compound according to any of claims 1 to 7 as an antibacterial agent for the prevention and/or inhibition of biofilm growth on surfaces.

17. The use of a compound according to any of claims 1 to 7 as an antibacterial agent for the prevention and/or inhibition of biofilm growth on medical appliances.

18. The use of a compound according to any of claims 1 to 7 as an antifouling agent.
